# EUROPEAN PATENT SPECIFICATION

(11) **EP 0 650 512 B1**
(45) Date of publication and mention of the grant of the patent: **24.10.2001**
(21) Application number: 93916993.4
(22) Date of filing: 06.07.1993
(51) Int. Cl.: C09J 189/00, A61K 38/38, A61L 24/10

(54) **ALDEHYDE-CURED PROTEINACEOUS ADHESIVE**
DURCH ALDEHYD GEHARTETERKLEBSTOFF AUF BASIS VON PROTEIN
COLLE PROTEIQUE DURCIE PAR UN ALDEHYDE

(30) Priority: 06.07.1992 US 908474
(43) Date of publication of application: 03.05.1995
(73) Proprietor: CryoLife, Inc., Kennesaw, GA 30144 (US)
(72) Inventor: Kowanko, Nicholas Dr., Moorehead, Minnesota 56560 (US)
(74) Representative: Gowshall, Jonathan Vallance
(86) International application number: PCT/US93/06375
(87) International publication number: WO 94/01508

(56) References cited:
- EP-A- 0 276 782
- EP-A- 0 388 220
- US-A- 2 597 228
- US-A- 3 395 106
- US-A- 4 359 049
- US-A- 4 431 757
- US-A- 4 492 684
- US-A- 4 627 879
- DATABASE WPI Week 9144 Derwent Publications Ltd., London, GB; AN 318142 & CN-A-1 049 456 (LAIYANG INST AGRIC) , 27 February 1991
- J. Cardiovasc. Surg., Vol. 31, 1990, (BACHET et al.), "Surgery of Type A Acute Aortic Dissection with Gelatine-Resorcine-Formol Biological Glue: A Twelve-Year Experience", pages 263-273, see the second paragraph on page 264, column 1.
- Encyclopedia of Polymer Science and Engineering, Vol. 1, "Adhesive Compositions", pages 547-577, see page 553, line 43 to page 554, line 46 (JOHN WILEY & SONS).
- DATABASE WPI, Week 8650, Derwent Publications Ltd., London, GB, Class , AN 329424 & JP 61246107 A

## Description

### Field of the Invention

This invention relates to adhesive compositions generally, and more particularly to tissue adhesives useful in the practice of medicine and surgery on human or animal subjects.

### Description of the Related Art

Various types of tissue adhesives are known in the art. Three of these, namely the cyanoacrylates, gelatin-formaldehyde compositions, and fibrin based glues, have received the most attention. For example, fibrin (a blood-clotting protein) and gelatin-formaldehyde have each been used in surgical adhesive applications, as have cyanoacrylates. Such adhesives work to a limited degree but have drawbacks as indicated below.

Several cyanoacrylates have been investigated for surgical use. For instance, some isobutyl cyanoacrylate formulations have been approved for veterinary use. Typically, monomer or a mixture of oligomers and/or monomer is applied to the site to be bonded where it rapidly polymerizes forming an adherent solid. One disadvantage of cyanoacrylate glues is that they require a dry field. Another is that the solid produced is non-absorbable, which limits the usefulness of the glue in internal applications. Also, the polymerization tends to be quite exothermic and adverse tissue response has been reported. See, for example, Bonutti, P. et al., *Clinical Orthopedics and Related Research,* Vol. 229, pages 241-248 (1988); Nelson, R. et al., *Arch. Surg.* Vol. 100, pages 295-298 (1970); and Celik, H., et al., "Nonsuture closure of arterial defect by vein graft using isobutyl-2 cyanoacrylate as a tissue adhesive." *Journal of Neurosurgical Sciences* Vol. 35, No. 2 (April-June 1991).

Glues based on gelatins that are cross-linked with formaldehyde have been used experimentally, principally in Europe, since about 1964. Of the several formulations that have been proposed, one that is referred to as "GRF" (gelatin, resorcinol, formol) is perhaps best known. Hot solutions of gelatin are mixed in situ with a curing agent consisting primarily of formaldehyde solution. The mixture rapidly sets to a solid which adheres to tissues. The chief objection to GRF glues has been the obligatory use of formaldehyde, a known hazardous material. Also, the gelatin must be applied hot, significantly above body temperature, and the techniques of mixing and application are quite critical for successful use of GRF. See, for example, Fabiani, et al., *Ann. Thorac. Surg.* 143-145 (1990); Fabiani, et al., *Supplement I Circulation* Vol. 80, No. 3 (September 1989); Bachet, et al., *J. Thorac. Cardiovasc. Surg.* Vol. 83 (1982), and Braunwald, et al., *Surgery* Vol. 59, No. 6 (June 1966).

Fibrin glues employ the natural processes of blood clot formation to generate an adhesive or sealant composition. One commercial product is "Tussicol®, available from Rugis, France. Another is "Fibrin Sealant Kit 1.0" available from Osterreichisches Institut for Haemoderivate, GMBH (subsidiary of Immuno AG, A-1220, Vienna, Austria). In the use of fibrin glues, two components are typically combined to form an artificial blood clot. One of the components is a solution of fibrinogen and blood clotting factors such as Factor XIII, and the other is primarily a solution of thrombin and calcium ion. Disadvantages of fibrin glues include their very low strength (generally less than 50 g/cm²) and relatively slow set up time. Also, the use of blood products (fibrinogen and co-factors) from multiple human donors presents an inherent risk of transmitting certain diseases to the patient. Procedures have been proposed for using autologous blood to prepare fibrin sealant. See, for instance, *Japanese Pat. No. 57149-229,* Issued September 1982.

EP-A-276,782 discloses the preparation of an enzyme electrode by application of a albumin solution containing a multifunctional aldehyde to the surface of an electroconductive substrate. The Encyclopedia of Polymer Science and Engineering Volume 1, 1985, pages 547-571 discloses various adhesive compositions including blood glues for use in plywood manufacture and veneering. US-A-4,431,757 discloses an adhesive comprising a polymer having an amide bond crosslinked to a dialdehyde compound for use in the lumber industry. US-A-4,359,049 discloses a twin-barrel syringe for applying a tissue adhesive.

### Summary of the Invention

The present invention provides an adhesive prepared by curing a composition comprising a protein component and a di- or polyfunctional aldehyde component. The cured adhesive preferably provides a bond that demonstrates a tear strength of at least about 75 g/cm² in a Tissue Adherence Test. In another embodiment, the invention provides a kit comprising protein and aldehyde components useful for the preparation of such a composition. In yet another embodiment the invention provides a tissue adhesive composition, per se, comprising protein and aldehyde components.

The adhesive of the present invention provides an optimal combination of such properties as strength, ease of use, tissue compatibility, and cost. A preferred adhesive of the invention is of high strength, stronger than sutures for example. The preferred composition is capable of creating a non-toxic bond in 30 seconds or less, and works well under even wet conditions.

The protein component of the present composition preferably comprises proteinaceous material of human or animal origin. Upon cross-linking the proteinaceous material with aldehyde, the resultant adhesive provides strong and rapid bonding to a wide range of substrates of natural or synthetic origin, providing a broad range of possible applications. Thus the composition of the present invention is capable of being cured to form an adhesive that can bond to living tissues, including muscle, skin, connective tissue, nerve tissue, vascular and cardiac tissue, adipose tissue, cartilage, bone, and the like, as well as to corresponding cadaver tissues, which may be preserved or otherwise chemically treated.

A strong bond can also be formed to natural or synthetic materials, e.g., to attach biocompatible materials, in order to attach them to other such materials as well as to tissue. Examples of suitable materials include leather and leather like materials, metals, and polymeric materials.

Examples of suitable leatherlike materials include coated and uncoated woven and unwoven fabrics (e.g., vinyl-coated or urethane-coated fabrics), and poromerics (i.e., "synthetic leather").

Examples of suitable metals include biocompatible metals such as nickel, silver, stainless steel, and aluminum, as well as metallized substrates such as inert polymeric materials coated with noble metals.

Examples of suitable polymeric materials include polymers such as poly(vinyl chloride), polyethylene, ultrahigh molecular weight polyethylene, polypropylene, polystyrene, silicone rubber, polycarbonate, polyester, polytetrafluoroethylene, fluorinated ethylene-propylene copolymers, polyolefin rubber, polyurethane, poly(vinyl alcohol), poly(acrylonitrile), polycacrylamide, polyacrolein, polysulfone, regenerated cellulose and cellulose acetate, poly(methyl methacrylate), methyl methacrylate-styrene copolymer, poly((hydroxyethyl methacrylate), and so on.

Such materials are typically used for the manufacture of permanent or disposable medical articles such as extracorporeal devices, catheters, tubing, prostheses (including vascular graft prostheses) and other artificial body parts and organs, membranes, surgical tapes, gauzes, bandages, and patches, sutures, shunts, cements, fixation devices, connectors and other medically useful components.

Examples of suitable materials useful include, for instance, woven dacron vascular prosthesis material (e.g., VERI-SOFT, DuPont); PTFE foam vascular prosthesis material (e.g., available from Gore Co.); gum rubber urinary catheter tubing; silicone rubber clad electrodes and leads for cardiac implantation (as available from Cardiac Pacemakers, Inc., St. Paul, MN).

Compositions of the present invention can be used, for instance, for the attachment of surgical grafts and devices, as well as for wound closure, trauma repair, hemostasis, and the like in the practice of human or veterinary medicine. Non-medical applications of the adhesive are also included within the scope of the invention.

In addition to the cured adhesive and related composition, the present invention further provides a method of preparing the adhesive composition; a method of adhering a plurality of tissue and/or biocompatible material surfaces together by use of the composition; and a combination comprising tissue surfaces adhered together by a cured adhesive of the present invention.

### Description of the Preferred Embodiments

The present invention provides an adhesive composition comprising an admixture of a plasma protein and a di- or polyfunctional aldehyde. In another embodiment, the invention provides an adhesive composition comprising an admixture of a globular protein and a di- or polyfunctional aldehyde, wherein a preferred globular protein is an albumin. Preferred adhesive compositions of the invention, when cured, have a tear strength of at least about 75 g/cm², and preferably at least about 400 g/cm², as measured in a Tissue Adherence Test.

A preferred adhesive composition is one in which the protein component is present at a concentration of between about 27% and about 53% by weight, based on the weight of the composition, and the aldehyde component is present at a concentration of between about 0.5% and about 5% by weight of the composition.

In another embodiment, the invention provides an adhesive compositions comprising a sterile admixture of a proteinaceous material and a di- or polyfunctional aldehyde, wherein the proteinaceous material is selected from the group consisting of a plasma protein and a globular protein.

In yet another embodiment, the invention provides a kit comprising a first container means having plasma or globular protein disposed therewithin and a second container means having a di- or polyfunctional aldehyde disposed therewithin. In a preferred embodiment, the kit comprises a first container means having a plasma protein component disposed therewithin in a concentration of between about 30% to 55% by weight, and a second container means comprising a di- or polyaldehyde component disposed therewithin in a concentration of between about 5% and about 15% by weight, the protein and aldehyde components being present in amounts sufficient to allow the mixing of one part by weight of aldehyde component with between 5 and 60 parts by weight of protein component.

In a related embodiment, the invention provides an applicator for delivering an adhesive composition to a tissue surface, the applicator having a proteinaceous material and a di- or polyfunctional aldehyde disposed therewithin. Preferably, the applicator is a syringe.

The applicator optionally comprises first and second separate compartments, the proteinaceous material being disposed within the first compartment and the aldehyde being disposed within the second compartment of the applicator.

A preferred adhesive of the present invention is prepared by forming, on or in the presence of a surface or surfaces to be bonded, a composition comprising:
Part A, comprising a protein present at a concentration of between about 27% and about 53% by weight of the composition; and
Part B, comprising a di- or polyaldehyde present at a concentration of between about 0.5% and about 5% by weight of the composition.

The preferred concentrations of ingredients can also be expressed in terms of their concentrations in the respective kit components, i.e., prior to the combination of those components to form a composition. When expressed in terms of kit components, kit component Part A preferably comprises protein at a concentration of between about 30% and about 55%, more preferably between about 35% and about 47%, and most preferably between about 40% and about 43% by weight, based on the weight of kit component Part A.

Similarly, kit component Part B preferably comprises a di- or polyaldehyde present at a concentration of between about 5% and about 15%, and preferably between about 10% and about 15% by weight of kit component Part B. The kit preferably contains Parts A and B in sufficient concentrations and amounts to allow mixing of one part of Part B with between about 5 to 60 parts of Part A, and preferably between about 20 to 60 parts of Part A, by weight.

Optionally, both Parts A and B can contain other, non-essential ingredients to make up the balance of the composition. Examples of such non-essential materials are neutral salts, carbohydrates, fibers and miscellaneous biological materials, wetting agents, antibiotics, preservatives, dyes, thickening agents, and the like. Upon mixing Parts A and B, the proteinaceous material in the plasma protein component is cross-linked by the aldehyde component in order to form a cured adhesive.

The final cross-linked cured adhesive is typically in the form of a water insoluble, rubbery or leathery proteinaceous solid that is substantially free of aldehydes. A suitable adhesive is typically adherent to the substrate to be bonded with a tear strength between about 75 g/cm² and about 800 g/cm², as determined in a Tissue Adherence Test, as described more fully in the EXAMPLES below. Preferred adhesives of the invention demonstrate a tear strength between about 100 g/cm² and about 500 g/cm², and particularly preferred are those that demonstrate a tear strength between about 250 g/cm² and about 400 g/cm². By comparison, typical tear strengths demonstrated by other materials in comparable Tissue Adherence Tests are as follows:

| Material | Tissue adherence (g/sq cm) |
|---|---|
| prolene suture (5-0) | about 300 |
| cyanoacrylate adhesives | 130 to 360 |
| "GRF" glue | 130 to 280 |
| fibrin sealants | 7 to 16 |
| bovine fibrinogen (6.7 wt%) | about 25 |

Bonding (i.e., mixing of the kit components in order to form a composition that cures to an adhesive) is achieved by combining the two part system (the parts being referred herein as Part A and B, respectively), and allowing the mixture to react on the surface or surfaces to be bonded. Bond formation is rapid, generally requiring less than one minute to complete. The resulting adhesion is strong, generally providing bonds with tear strengths of about 400 g/cm² to about 600 g/cm². The term "tear strength" will be used interchangeably herein with the term "tissue adherence", as determined by the Tissue Adherence Test described herein. Tear strengths of 1300 g/cm² and more have been obtained by using this invention. The upper limits of tear strength have not been determined, and they are not intended to be interpreted as limiting the invention.

Part A of the presently claimed composition preferably comprises an aqueous solution containing between about 30% and about 55% by weight of a plasma protein component. At concentrations below about 30%, the cured product is more likely to form curds in suspension, rather than a homogeneous mass, and adhesive properties will be poor. At concentrations greater than about 55%, the composition tends to cure too quickly when the protein and aldehyde components are mixed. Concentrations, as expressed herein, are based on the lyophilized weight of the various solid components described.

In a preferred embodiment, kit component Part A comprises between about 35% and about 55% by weight of the plasma protein component, and in particularly preferred embodiment, between about 40% and about 43% by weight. The balance of Part A is typically an inert vehicle, for instance, water, dilute buffer, and/or saline solution, optionally containing non-essential materials which do not significantly interfere with the adhesive forming reaction. Examples of such non-essential materials are neutral salts, carbohydrates, fibers and miscellaneous biological materials, wetting agents, antibiotics, preservatives, dyes, thickening agents, and the like. For example, non-essentials such as fibrinogen or poly(ethylene glycol) as shown in EXAMPLES 5 and 19 and in 9 respectively, hereof may be included.

Kit component Part B, used to prepare the presently claimed composition, preferably comprises a solution of a di-or polyaldehyde in water or other suitable medium. Glutaraldehyde as a 5% to 15% (and preferably a 10% to 15%) solution is preferred, but other aldehyde materials are also suitable. For instance, where the aldehyde component comprises a dialdehyde, suitable dialdehydes are selected from the group consisting of glyoxal, succinaldehyde, malealdehyde, and phthalaldehyde, in addition to glutaraldehyde. Thus, aqueous glyoxal is satisfactory for Part B, as are aqueous mixtures of di-and polyaldehydes obtained by oxidative cleavage of carbohydrates and their derivatives with periodate, ozone, or the like. Other water soluble di- and/or polyaldehydes will be readily recognized as useful for the purpose of this invention.

When a suitable mixture of Parts A and B is allowed to react on a surface or surfaces to be bonded, an adherent composition forms, generally in less than one minute, and often within 30 seconds or less. A wide range of substrates can be bonded by this process, as previously indicated. No significant exotherm is observed, and good adhesion is obtained to wet surfaces, thus providing a wide ange of usefulness for the invention.

As a surgical adhesive, the preferred composition of the invention provides a cured adhesive that has a bonding strength that is many times stronger than conventional fibrin adhesives and traditional sutures. Several experimental surgical procedures were successfully carried out (in a porcine model) using the adhesive composition. For instance, a perforated aorta and bowel were repaired by gluing patch materials to the lesions, and hemostasis was quickly achieved in a resected spleen by application of the adhesive.

As previously stated, the usefulness of the claimed composition is provided by the mixing of kit components Parts A and B. The properties of the composition and resultant adhesive are determined largely by the protein type and content of Part A and the type and weight ratio of aldehyde component in Part B. The manner in which the bonding composition is actually mixed in practice (i.e., immediately prior to cure) is not a limiting factor, and may vary with the intended use. Preferred ranges of concentrations for Parts A and B which on mixing produce the bonding composition will be discussed below as well as in the

### EXAMPLES.

As described above, Part A of the adhesive composition preferably comprises a substantially aqueous solution of either ovalbumin or a plasma protein component, e.g., proteinaceous material of human or animal origin. A preferred protein source for use as Part A is a plasma protein selected from the group consisting of concentrated serum, serum albumin, and combinations thereof. Albumins including ovalbumins are preferred proteins, and serum albumins of human or other animal origin are particularly preferred.

The proteinaceous material may be provided as a purified protein or in a mixture in which the proteins such as serum albumins are the predominant ingredients. For instance, the presence of a small amount of the plasma component fibrinogen (e.g., between about 1% and 20%, and preferably between about 1% and about 10%, by weight of Part A) , has been found to improve the adhesive properties of a cured composition of the present invention. For example, the solid mixtures obtained by dehydration of blood plasma or serum, or of commercial solutions of stabilized plasma proteins, can be used to prepare Part A. These mixtures, generally referred to as plasma solids or composite to serum solids, typically contain albumins as their major ingredients, of the order of about 50% to about 90% by weight. As used herein, the term "plasma" refers to whole blood from which the corpuscles have been removed by centrifugation. The term "serum" refers to plasma that has additionally been treated to prevent agglutination by removal of its fibrinogen and or fibrin, or by inhibiting the fibrin clot formation through addition of reagents such as citrate or EDTA.

Part B of the adhesive composition preferably comprises a substantially aqueous solution of di- or polyaldehydes. A wide range of these substances exist, and their usefulness is restricted largely by availability and by their solubility in water. For example, aqueous glyoxal (ethandial) is useful, as is aqueous glutaraldehyde (pentandial). Water soluble mixtures of di- and polyaldehydes prepared by oxidative cleavage of appropriate carbohydrates with periodate, ozone, or the like are also useful. Glutaraldehyde is the preferred dialdehyde ingredient of Part B.

When Parts A and B are brought together as described in the Examples hereinbelow, the resultant product rapidly hardens to a strong leathery or rubbery material within a short time of mixing, generally on the order of about 15 to about 30 seconds. Strong adhesion results if the adhesive composition is present on one or both surfaces to be bonded and the surfaces are brought together before the bond hardens. Full bond strength is generally attained in less than a minute but those familiar with this art will recognize that manipulating variables such as the particular ingredients used as Parts A and B, temperature, and so forth can be used to affect the rate of bond formation. For instance, markedly reduced cure rates are observed when di- or polyaldehydes derived by oxidative cleavage or carbohydrates are used as Part B. Likewise, when Part A is based on plasma solids, full bond strength is generally obtained about twice as fast as when purified bovine serum albumin is used.

As described herein, effective bonding results when a preferred composition including Parts A and B falls within the acceptable range specified above, that is, between about 27% and about 53% by weight of protein, as well as the requisite amounts of dialdehyde and water. However, if the composition is to be produced by mechanically mixing Parts A and B on the surface(s) to be bonded, there are some preferred ranges of concentration within which Parts A and B shoped fall to facilitate the mixing. Thus, it is preferred to use protein solutions (Part A) with a solids content of between about 30% and about 50% by weight of Part A, and to mix these with dialdehyde solutions (Part B) of between about 5% and about 15% by weight of Part B.

When generating the adhesive compositions of this invention, the Parts A and B can be pre-mixed and applied to the surface(s) to be bonded immediately following mixing by means of a syringe, catheter or other device. Alternatively, the Parts A and B can be applied simultaneously, as for example from a dual nozzle device and mixed on the surface(s) to be bonded. Sequential application of Parts A and B to the surfaces is also satisfactory, with the sequence B, and A, then B preferred.

In a particularly preferred embodiment it is most convenient to mix Part A (containing between about 40% and about 43%, by weight, plasma proteins) with Part B (containing between about 5% and about 15% by weight glutaraldehyde or other di- or polyaldehyde). However, in some applications it may be preferable to deviate from the above concentrations of A and B, provided, however, that the composition of the resulting mixture remains within the limits specified earlier. It is also convenient in some applications to use a slight excess of B, since unreacted material can be readily neutralized after the bond is formed. Dilute solutions of proteins, peptides, or amino acids, for instance, applied at about 5% concentration are suitable for the purpose of neutralizing excess aldehyde component.

In their preferred embodiments, kit component Part A is typically a pasty material and Part B is typically liquid. When combined, a honey-like composition (in terms of both appearance and consistency) is preferably obtained.

In addition to the presently described cured adhesive and related kit and composition of the present invention, the present invention further provides a method of preparing a tissue adhesive comprising the steps of (a) providing a composition as described herein comprising a protein component and a di- or polyfunctional aldehyde component, and (b) curing the composition to form an adhesive. Preferably the protein component is a plasma protein and the cured composition is capable of providing a bond that demonstrates a tear strength of at least about 75 g/cm² in a Tissue Adherence Test.

Part A can be prepared by dissolving in water the dry proteinaceous solids, optionally containing non-essential materials, or the solids may be dissolved in water which may optionally contain said non-essentials. The dry proteinaceous solids may be prepared by lyophilization of dilute proteinaceous solutions such as plasma or serum, or of commercial plasma extenders such as Plasma-Plex® (Plasma-Plex Plasma protein fraction (human) USP 5% solution, heat treated, Armour Pharmaceutical Company, Kankakee, Illinois 60901) or Plasmanate® (Plasma protein fraction (human) USP 5% solution Miles, Inc. Cutter Biological, Elkhart, Indiana 46515, USA, Miles Canada, Inc., Etobiocoke, Ontario Canada), which are stabilized, reconstituted solutions of plasma proteins containing about 5% protein by weight, or of other appropriate solutions. Purified protein powders such as human or animal serum albumins, ovalbumin, or mixtures of protein powders may also serve to prepare Part A.

An alternative route to the preparation of Part A is the concentration of dilute proteinaceous solutions to the required solids content. This approach is particularly useful for the preparation of adhesive from a patient's own blood or from screened blood obtained from a single donor. Many techniques are available for concentrating protein solutions and they are well known by those skilled in the art. Among them may be listed lyophilization and reconstitution (mentioned above), evaporation, contact with hygroscopic solids, dialysis, ultrafiltration, and vacuum centrifugal evaporation. Other methods of concentrating dilute solutions may be preferable in particular applications.

In addition to the presently escribed cured adhesive and related composition of the present invention, the present invention further provides a method of adhering a plurality of tissue and/or tissue-contacting surfaces together by use of the composition. The method comprises the steps of (a) applying to one or more tissue surfaces a composition comprising a protein component and a di- or polyfunctional aldehyde component, the composition being capable of being cured to provide an adhesive bond, (b) contacting the surfaces, and (c) curing the composition in order to adhere the surfaces. Preferably the protein component is a plasma protein and the cured composition demonstrates a tear strength of at least about 75 g/cm² in a Tissue Adherence Test. In the practice of the method the components of the composition can be applied sequentially to the surface and mixed together in situ to form the composition. In the alternative, the components can be mixed together to form the composition prior to its application to the tissue surface.

The invention therefore provides, in one aspect, a method of bonding a biological tissue to a substance comprising contacting the tissue and the substance with an adhesive composition comprising an admixture of a protein and a di- or polyfunctional aldehyde under conditions such that a bond between the tissue and substance is formed. The substance can be a biological tissue or a synthetic material.

In the method of the present invention the protein and aldehyde can be applied sequentially to the surface and mixed together in situ to form the composition. Alternatively, the protein and aldehyde can be mixed together to form the composition prior to its application to the tissue surface.

In addition to the presently described cured adhesive and related kit and composition of the present invention, the present invention further provides a combination comprising one or more tissue surfaces adhered together, optionally including one or more biocompatible device surfaces, by a cured adhesive of the present invention. The combination comprises a plurality of surfaces adhered together by a cured adhesive prepared from a composition comprising a protein component and a di- or polyfunctional aldehyde component. Preferably the protein component comprises a plasma protein and the composition is capable of being cured to provide a bond test that demonstrates a tear strength of at least about 75 g/cm² in a Tissue Adherence Test.

Preferred embodiments of the invention are further illustrated by the following EXAMPLES:

### EXAMPLE 1

Dry plasma solids were obtained by lyophilizing fresh frozen human plasma. Water was added to this solid to produce a viscous solution containing 45% of solids by weight. This solutions was used as Part A. Part B was aqueous glutaraldehyde solution containing 10% of glutaraldehyde by weight. Two rectangular blocks of meat were sprayed lightly with B on the surfaces to be bonded. The surfaces were then coated with A to a thickness of about 1mm to about 2mm, and again sprayed with B. The ratio of A to B was 7 to 1 by weight. The surfaces were joined within about 10 seconds of the application of A and held in position until cure was complete, generally 15-60 seconds, depending on temperature and on the effectiveness of mixing A and B.

### Tissue Adherence Test

To determine tear strength the glued rectangular block of meat was attached with clamps to a spring balance at one end and to a variable weight on the other. The weight was increased progressively until the bond or adjoining meat broke. Tear strength was recorded in g/cm² required to break the bond. Tear strengths were determined one minute after joining the surfaces, unless otherwise noted. Typical tear strengths for the above composition were 445 g/cm². The strength of the meat itself serves to determine an upper limit of tissue adherence that can be determine using such a test. In this particular test, that upper limit is approximately 1200 g/cm² (identified as "1200+ g/cm²"). Although the upper limit in any particular test will depend, for instance, on the type of meat, the direction of cut, and so on, the values obtained below those upper limits are comparable between various samples. Although such strong adhesives might be of less utility for use with soft tissue adhesion, they are particularly useful in such applications as tendon and connective tissue adhesion.

When the sequence of application of A and B was reversed or when A and B were applied simultaneously or when A and B were pre-mixed immediately prior to application, essentially the same bond strengths were observed.

### EXAMPLE 2

Same as EXAMPLE 1, except that a heat treated 5% plasma protein fraction (human) USP was used to produce the dry solids, and its concentration in Part A was 47% by weight. Adhesion was achieved as described above. Tear strengths ranged from 550-624 g/cm².

### EXAMPLE 3

Bovine serum albumin was dissolved in water to produce a solution containing 40% by weight solids, which was then used as Part A as described in EXAMPLE 1 to bond meat blocks. Tear strengths ranged from 514-629 g/cm².

### EXAMPLE 4

Human serum albumin solutions containing 40-45% by weight of protein were prepared by concentrating 25% solutions by means of dialysis. When these solutions were used as Part A in bonding as described above, the tear strengths ranged from 605-922 g/cm².

### EXAMPLE 5

When Part A was a solution with a solids content of 45.5% by weight and consisting of a mixture of bovine serum albumin and bovine fibrinogen in which the albumin constituted 90-97%, the resulting bond tear strengths ranged from 786 to over 1280 g/cm².

### EXAMPLE 6

Synthetic vascular prostheses made of a Dacron(TM) material and prostheses made of Teflon(TM) material, as well as cadaver derived human vascular graft materials were successfully bonded to meat or to each other using any of the compositions described in all of the EXAMPLES included herein. For instance, the tear strength obtained with the composition of EXAMPLE 5 for the bond between beef and woven Dacron vascular prosthesis was above 890 g/cm². measured in shear mode while that to a Teflon graft was 670 g/cm². Similarly, implantable medical electrodes coated with silicone rubber showed a tear strength of 1080 g/cm² using bovine serum albumin in Part A.

### EXAMPLE 7

Part A, containing 30-32% by weight of human serum albumin was prepared from either fresh plasma or from stabilized 5% plasma protein solutions by ultrafiltration. When 50% aqueous glutaraldehyde was used as component B the tear strengths of the resulting bonds ranged between 267-335 g/cm².

### EXAMPLE 8

Part A was 30% by weight of human plasma protein prepared from 5% stabilized solution (USP) as described in EXAMPLE 7 above. Part B was 25% by weight aqueous glutaraldehyde. Parts A and B were mixed in a weight ratio of 10 Parts A to 1 Part of B resulting in a mixture containing 27% proteins by weight. This mixture was immediately applied to the surfaces of two meat blocks and the blocks were joined. The resulting bond had a tear strength of 75 g/cm².

### EXAMPLE 9

When meat blocks were joined as described in EXAMPLE 1, but using 47% aqueous bovine serum albumin as A and 10% aqueous glyoxal as B the tear strength was 333 g/cm².

### EXAMPLE 10

A specimen containing a high proportion of skin and adipose tissue was joined using as Part A an aqueous solution containing about 41% bovine serum albumin and 4% polyethylene glycol) of 15,000 to 20,000 molecular weight, and 10% glutaraldehyde as Part B. The tear strength of the bond produced was 1300 g/cm².

### EXAMPLE 11

The adhesive composition described in EXAMPLE 1 was successfully used in several surgical procedures on a 35-40 pound pig. The procedures carried out were applications of patches (without sutures) to perforated abdominal aorta and bowel, and hemostasis of resected spleen. The patches were cut from human cadaver pulmonary artery tissue processed for use as a graft material in human cardiovascular surgery.

### EXAMPLE 12

Part A was 47% bovine serum albumin. Part B was prepared as follows: methyl glucoside (0.5 g) was dissolved in water (4.1 ml) and oxidized with sodium periodate (1.0 g) in the presence of sodium bicarbonate in an ice bath during 45 min. The products of this reaction are known to be a complex dialdehyde in about 10% concentration by weight, as well as non essential salts. This solution was used as Part B without purification. The mixture cured to a flexible bond in about one hour with a tear strength of 320 g/cm².

### EXAMPLE 13

Same as EXAMPLE 11, but using trehalose (0.5 g), sodium periodate (1.2 g) and sodium bicarbonate (0.5 g) as reagents dissolved in water (4.0 ml) in an ice bath to produce Part B. This product is known to contain a mixture of complex di-, tetra- and polymeric aldehydes in about 10% solution, along with non-essential salts. Tear strength after one hour was 245 g/cm².

### EXAMPLE 14

As in EXAMPLE 11, but using sucrose (0.5 g), sodium periodate (1.0 g), sodium bicarbonate (1.0 g) and water (5.0 ml). The resulting product solution is known to contain a mixture of complete di-, tetra- and polyaldehydes in about 8%. Resulting bond strength was 364 g/cm².

### EXAMPLE 15

The solid mixture obtained by lyophilizing egg white, and consisting predominantly of ovalbumin, was dissolved in water to make solutions with a solids content ranging from 44.0-44.8%. When these solutions were used as Part A in adhesion studies using 10% aqueous glutaraldehyde as Part B, the tear strengths obtained ranged from 255-339 g/cm².

### EXAMPLE 16

Bovine serum albumin (BSA) was dissolved in water in the concentrations shown and used as Part A. Part B was 10% aqueous glutaraldehyde. The following tear strengths were measured:

| Weight % BSA | Tear Strength (gm/cm²) |
|---|---|
| 25 | <50 |
| 30 | <50 |
| 35 | 100 |
| 40 | 514-629 |
| 45 | 610 |
| 50 | 96-557* |

| | |
|---|---|
| *Too viscous to spread evenly. | |

### EXAMPLE 17

Plasma-Plex® , a 5% solution of human protein fraction (USP) was lyophilized, the resulting solids constituted with water to the concentrations shown below, and the solutions used as Part A. Part B was 10% aqueous glutaraldehyde. The following tear strengths were observed:

| Weight % HSA | Tear Strength (gm/cm²) |
|---|---|
| 35 | <50 |
| 37 | 163-257 |
| 41 | 250-381 |
| 43 | 358-413 |
| 45 | 423-567⁺ |
| 47 | 550-624 |
| 51 | 400-527⁺ |
| 53 | 338-500 |
| 55 | 85-390* |

| | |
|---|---|
| ⁺Indicates that the substrate tissue tore rather than the bond. | |
| *Too viscous to apply evenly. | |

### EXAMPLE 18

As in EXAMPLE 17, but using solids obtained by lyophilizing fresh frozen plasma gave the following tear strengths:

| Weight % HSA | Tear Strength (gm/cm²) |
|---|---|
| 30 | 136-269 |
| 35 | 486 |
| 40 | 550⁺ |
| 45 | 544-445₊ |
| 50 | 278-361 |
| 55 | 150-226* |
| 60 | -Not soluble- |

| | |
|---|---|
| ⁺Indicates substrate tissue tore rather than the bond. | |
| *Too viscous to spread well. | |

### EXAMPLE 19

Mixtures of proteins can be used in the concentration ranges of the invention for Part A. For example, bovine serum albumin (BSA) and bovine fibrinogen (BF) produce an excellent adhesive: Total solids (mixed) 45.5 weight percent.

| PERCENT BSA | PERCENT BF | Tear Strength (gm/cm²) |
|---|---|---|
| 97 | 3 | 708-1280⁺ |
| 93.3 | 6.7 | 797⁺ |
| 90 | 10 | 786 |
| 80 | 20 | 736-237* |

| | | |
|---|---|---|
| ⁺Indicates substrate tissue tore rather than the bond. | | |
| *BF not fully soluble, solution is rubbery. | | |

Mixtures such as these, for instance having 93 parts BSA to 7 parts BF, at 45-46% solids, particularly bonded aggressively to Dacron(TM) and PTFE at strengths of 890⁺ to 1200⁺.

Generally, good bonding is achieved either by applying A or B first or simultaneously e.g., from a dual nozzle. Also, methods of mixing in situ provide good bonds. Parts A and B may be applied in sequence, B then A, then B; or A, then B; or simultaneously with mixing on the surface(s) to be bonded; or through a catheter or catheters; or via syringe or similar device immediately following premixing.

## Claims

1. An adhesive composition comprising an admixture of a plasma protein or a globular protein and di- or polyfunctional aldehyde for use as a medical adhesive, wherein the composition does not comprise chlorohexidine digluconate and does not comprise insulin.

2. An adhesive composition according to Claim 1, wherein the protein comprises an albumin.

3. An adhesive composition according to Claims 1 or 2 wherein the adhesive, when cured, has a tear strength of at least 75g/cm² as measured in a Tissue Adherence Test.

4. An adhesive composition according to Claim 3 wherein the tear strength is at least 400 g/cm².

5. An adhesive composition according to any one of Claims 1 to 4, wherein the protein component is present at a concentration of between 27% and 53% by weight, based on the weight of the composition.

6. An adhesive composition according to any one of Claims 1 to 5 wherein the aldehyde component is present at a concentration of between 0.5% and 5% by weight of the composition.

7. An adhesive composition according to any one of Claims 1 to 6 wherein the aldehyde component is a dialdehyde selected from the group consisting of glyoxal, succinaldehyde, glutaraldehyde, malealdehyde and phthalaldehyde.

8. The adhesive composition of Claim 7 wherein the aldehyde is glutaraldehyde.

9. An adhesive composition according to any one of the preceding claims wherein the admixture is sterile.

10. A sterile medical adhesive composition according to any one of Claims 1 to 9, comprising an admixture of a globular protein and a di- or polyfunctional aldehyde wherein the adhesive, when cured, has a tear strength of at least 75 g/cm² as measured in a Tissue Adherence Test.

11. A kit comprising a first container means having plasma protein or globular protein disposed therewithin and a second container means having a di- or polyfunctional aldehyde disposed therewith for use in the therapeutic administration of a medical adhesive comprising an admixture of a plasma protein or a globular protein and di- or polyfunctional aldehyde.

12. A kit according to Claim 11 wherein the protein is an albumin.

13. A kit comprising a first container means having a plasma protein component disposed therewithin in a concentration of between 30% to 55% by weight, and a second container means comprising a di- or polyaldehyde component disposed therewithin in a concentration of between 5% and 15% by weight, the protein and aldehyde components being present in amounts sufficient to allow the mixing of one part by weight of aldehyde component with between 5 and 60 parts by weight of protein component for use in the therapeutic administration of a medical adhesive comprising an admixture of a plasma protein or a globular portion and di- or polyfunctional aldehyde.

14. An applicator for delivering an adhesive composition to a tissue surface, the applicator having a plasma protein or a globular protein and a di- or polyfunctional aldehyde disposed therewithin wherein the applicator comprises first and second separate compartments, the protein being disposed within the first compartment and the aldehyde being disposed within the second compartment of the applicator.

15. An applicator according to Claim 14 wherein the applicator is a syringe.

16. An adhesive composition according to any one of Claims 1 to 10 for use in a method of bonding a biological tissue to a substance comprising contacting the tissue and the substance.

17. An adhesive according to Claim 16 wherein the substance is a biological tissue.

18. An adhesive according to Claim 16 or 17 wherein the substance is a synthetic material.

19. An adhesive according to Claim 16, 17 or 18, wherein the protein and aldehyde are applied sequentially to the surface and mixed together *in situ* to form the composition.

20. An adhesive according to Claim 16, 17 or 18 wherein the protein and aldehyde are mixed together to form the composition prior to its application to the tissue surface.

21. Use of a composition comprising an admixture of a plasma protein or a globular protein and di- or polyfunctional aldehyde for the manufacture of a medical adhesive.

22. Use according to Claim 21, wherein the protein is an albumin.

23. Use according to Claims 21 or 22 wherein the adhesive, when cured, has a tear strength of at least 75g/cm² as measured in a Tissue Adherence Test.

24. Use according to Claim 23 wherein the tear strength is at least 400 g/cm².

25. Use according to any one of Claims 21 to 24, wherein the protein component is present at a concentration of between 27% and 53% by weight, based on the weight of the composition.

26. Use according to any one of Claims 21 to 25 wherein the aldehyde component is present at a concentration of between 0.5% and 5% by weight of the composition.

27. Use according to any one of Claims 21 to 26 wherein the aldehyde component is a dialdehyde selected from the group consisting of glyoxal, succinaldehyde, glutaraldehyde, malealdehyde and phthalaldehyde.

28. Use of Claim 27 wherein the aldehyde is glutaraldehyde

29. Use according to any one of Claims 21 to 28 wherein the admixture is sterile.

30. Use according to any one of Claims 21 to 29, comprising an admixture of a globular protein and a di- or polyfunctional aldehyde wherein the adhesive, when cured, has a tear strength of at least 75 g/cm² as measured in a Tissue Adherence Test.

31. Use according to any one of Claims 21 to 30 for use in a method of bonding a biological tissue to a substance comprising contacting the tissue and the substance.

32. Use according to Claim 31 wherein the substance is a biological tissue.

33. Use according to Claim 31 or 32 wherein the substance is a synthetic material.

34. Use according to Claim 31, 32 or 33, wherein the protein and aldehyde are applied sequentially to the surface and mixed together *in situ* to form the composition.

35. Use according to Claim 31, 32 or 33 wherein the protein and aldehyde are mixed together to form the composition prior to its application to the tissue surface.

## Patentansprüche

1. Klebstoffzusammensetzung, die eine Mischung aus einem Plasmaprotein oder einem globulären Protein und di- oder polyfunktionellem Aldehyd umfaßt, zur Verwendung als ein medizinischer Klebstoff, wobei die Zusammensetzung nicht Chlorhexidin-Digluconat umfaßt und nicht Insulin umfaßt.

2. Klebstoffzusammensetzung nach Anspruch 1, **dadurch gekennzeichnet, daß** das Protein ein Albumin umfaßt.

3. Klebstoffzusammensetzung nach den Ansprüchen 1 oder 2, **dadurch gekennzeichnet, daß** der Klebstoff, wenn er ausgehärtet ist, eine Reißfestigkeit von wenigstens 75 g/cm², gemessen in einem Tissue Adherence Test, aufweist.

4. Klebstoffzusammensetzung nach Anspruch 3, **dadurch gekennzeichnet, daß** die Reißfestigkeit wenigstens 400 g/cm² beträgt.

5. Klebstoffzusammensetzung nach einem der Ansprüche 1 bis 4, **dadurch gekennzeichnet, daß** die Protein-Komponente in einer Konzentration von zwischen 27 und 53 Gew.-%, bezogen auf das Gewicht der Zusammensetzung, vorliegt.

6. Klebstoffzusammensetzung nach einem der Ansprüche 1 bis 5, **dadurch gekennzeichnet, daß** die Aldehyd-Komponente in einer Konzentration zwischen 0,5 und 5 Gew.-% der Zusammensetzung vorliegt.

7. Klebstoffzusammensetzung nach einem der Ansprüche 1 bis 6, **dadurch gekennzeichnet, daß** die Aldehyd-Komponente ein Dialdehyd ist, das ausgewählt ist aus der Gruppe, die aus Glyoxal, Succinaldehyd, Glutaraldehyd, Malealdehyd und Phthalaldehyd besteht.

8. Klebstoffzusammensetzung nach Anspruch 7, **dadurch gekennzeichnet, daß** das Aldehyd Glutaraldehyd ist.

9. Klebstoffzusammensetzung nach einem der vorangehenden Ansprüche, **dadurch gekennzeichnet, daß** die Mischung steril ist.

10. Sterile medizinische Klebstoffzusammensetzung nach einem der Ansprüche 1 bis 9, die eine Mischung aus einem globulären Protein und einem di- oder polyfunktionellen Aldehyd umfaßt, wobei der Klebstoff, wenn er ausgehärtet ist, eine Reißfestigkeit von wenigstens 75 g/cm², gemessen in einem Tissue Adherence Test, aufweist.

11. Kit, welcher einen ersten Behälter, in dem Plasmaprotein oder globuläres Protein enthalten ist, und einen zweiten Behälter, in dem ein di- oder polyfunktionelles Aldehyd enthalten ist, umfaßt, zur Verwendung bei der therapeutischen Verabreichung eines medizinischen Klebstoffes, der eine Mischung aus einem Plasmaprotein oder einem globulären Protein und di- oder polyfunktionellem Aldehyd umfaßt.

12. Kit nach Anspruch 11, **dadurch gekennzeichnet, daß** das Protein ein Albumin ist.

13. Kit, welcher einen ersten Behälter, in dem eine Plasmaprotein-Komponente in einer Konzentration von zwischen 30 bis 55 Gew.-% enthalten ist, und einen zweiten Behälter, in dem eine Di- oder Polyaldehyd-Komponente in einer Konzentration von zwischen 5 und 15 Gew.-% enthalten ist, umfaßt, wobei die Protein- und Aldehyd-Komponenten in ausreichenden Mengen vorliegen, um das Vermischen von einem Gewichtsteil Aldehyd-Komponente mit zwischen 5 und 60 Gewichtsteilen Protein-Komponente zu ermöglichen, zur Verwendung bei der therapeutischen Verabreichung eines medizinischen Klebstoffes, der eine Mischung aus einem Plasmaprotein und einem globulären Protein und di- oder polyfunktionellem Aldehyd umfaßt.

14. Applikator für das Aufbringen einer Klebstoffzusammensetzung auf eine Gewebeoberfläche, wobei in dem Applikator ein Plasmaprotein oder ein globuläres Protein und ein di- oder polyfunktionelles Aldehyd enthalten sind, wobei der Applikator erste und zweite getrennte Abteilungen umfaßt, wobei das Protein in der ersten Abteilung und das Aldehyd in der zweiten Abteilung des Applikators enthalten ist.

15. Applikator nach Anspruch 14, **dadurch gekennzeichnet**, **dadurch gekennzeichnet, daß** der Applikator eine Spritze ist.

16. Klebstoffzusammensetzung nach einem der Ansprüche 1 bis 10 zur Verwendung in einem Verfahren zum Binden eines biologischen Gewebes an eine Substanz, welches das In-Kontakt-Bringen des Gewebes und der Substanz umfaßt.

17. Klebstoff nach Anspruch 16, **dadurch gekennzeichnet, daß** die Substanz ein biologisches Gewebe ist.

18. Klebstoff nach Anspruch 16 oder 17, **dadurch gekennzeichnet, daß** die Substanz ein synthetisches Material ist.

19. Klebstoff nach Anspruch 16, 17 oder 18, **dadurch gekennzeichnet, daß** das Protein und Aldehyd nacheinander auf die Oberfläche aufgebracht und in situ miteinander vermischt werden, um die Zusammensetzung zu bilden.

20. Klebstoff nach Anspruch 16, 17 oder 18, **dadurch gekennzeichnet, daß** das Protein und Aldehyd vermischt werden, um die Zusammensetzung vor ihrem Aufbringen auf die Gewebeoberfläche zu bilden.

21. Verwendung einer Zusammensetzung, die eine Mischung aus einem Plasmaprotein oder einem globulären Protein und di- oder polyfunktionellem Aldehyd umfaßt, zur Herstellung eines medizinischen Klebstoffes.

22. Verwendung nach Anspruch 21, **dadurch gekennzeichnet, daß** das Protein ein Albumin ist.

23. Verwendung nach den Ansprüchen 21 oder 22, **dadurch gekennzeichnet, daß** der Klebstoff, wenn er ausgehärtet ist, eine Reißfestigkeit von wenigstens 75 g/cm², gemessen in einem Tissue Adherence Test, aufweist.

24. Verwendung nach Anspruch 23, **dadurch gekennzeichnet, daß** die Reißfestigkeit wenigstens 400 g/cm² beträgt.

25. Verwendung nach einem der Ansprüche 21 bis 24, **dadurch gekennzeichnet, daß** die Protein-Komponente in einer Konzentration von zwischen 27 und 53 Gew.-%, bezogen auf das Gewicht der Zusammensetzung, vorliegt.

26. Verwendung nach einem der Ansprüche 21 bis 25, **dadurch gekennzeichnet, daß** die Aldehyd-Komponente in einer Konzentration von zwischen 0,5 und 5 Gew.-% der Zusammensetzung vorliegt.

27. Verwendung nach einem der Ansprüche 21 bis 26, **dadurch gekennzeichnet, daß** die Aldehyd-Komponente ein Dialdehyd ist, das ausgewählt ist aus der Gruppe, die aus Glyoxal, Succinaldehyd, Glutaraldehyd, Malealdehyd und Phthalaldehyd besteht.

28. Verwendung nach Anspruch 27, **dadurch gekennzeichnet, daß** das Aldehyd Glutaraldehyd ist.

29. Verwendung nach einem der Ansprüche 21 bis 28, **dadurch gekennzeichnet, daß** die Mischung steril ist.

30. Verwendung nach einem der Ansprüche 21 bis 29, umfassend eine Mischung aus einem globulären Protein und einem di- oder polyfunktionellen Aldehyd, wobei der Klebstoff, wenn er ausgehärtet ist, eine Reißfestigkeit von wenigstens 75 g/cm², gemessen in einem Tissue Adherence Test, aufweist.

31. Verwendung nach einem der Ansprüche 21 bis 30, zur Verwendung in einem Verfahren zum Binden eines biologischen Gewebes an eine Substanz, welches das In-Kontakt-Bringen des Gewebes und der Substanz umfaßt.

32. Verwendung nach Anspruch 31, **dadurch gekennzeichnet, daß** die Substanz ein biologisches Gewebe ist.

33. Verwendung nach Anspruch 31 oder 32, **dadurch gekennzeichnet, daß** die Substanz ein synthetisches Material ist.

34. Verwendung nach Anspruch 31, 32 oder 33, **dadurch gekennzeichnet, daß** das Protein und Aldehyd nacheinander auf die Oberfläche aufgebracht und in situ miteinander vermischt werden, um die Zusammensetzung bilden.

35. Verwendung nach Anspruch 31, 32 oder 33, **dadurch gekennzeichnet, daß** das Protein und Aldehyd vermischt werden, um die Zusammensetzung vor ihrem Aufbringen auf die Gewebeoberfläche zu bilden.

## Revendications

1. Composition adhésive comprenant un mélange d'une protéine plasmatique ou d'une protéine globulaire ou d'un aldéhyde polyfonctionnel ou d'un dialdéhyde pour être utilisée comme adhésif médical, dans laquelle la composition ne comprend pas de digluconate de chlorhexidine ni d'insuline.

2. Composition adhésive selon la revendication 1, dans laquelle la protéine comprend de l'albumine.

3. Composition adhésive selon les revendications 1 ou 2, dans laquelle l'adhésif, lorsqu'il a durci, présente une résistance au déchirement au moins égale à 75 g/cm², tel que mesuré grâce au Test d'Adhérence des Tissus.

4. Composition adhésive selon la revendication 3, dans laquelle la résistance au déchirement est au moins égale à 400 g/cm².

5. Composition adhésive selon l'une quelconque des revendications 1 à 4, dans laquelle la composante protéinique est présente a une concentration comprise entre 27 % et 53 % en poids, par rapport au poids de la composition.

6. Composition adhésive selon l'une quelconque des revendications 1 à 5, dans laquelle la composante aldéhydique est présente à une concentration comprise entre 0,5 % et 5 % en poids, par rapport au poids de la composition.

7. Composition adhésive selon l'une quelconque des revendlcations 1 à 6, dans laquelle la composante aldéhydique est un dialdéhyde choisi parmi le groupe composé de glyoxal, succinaldéhyde, glutaraldéhyde, maléaldéyde et phtalaldéhyde.

8. Composition adhéslve selon la revendication 7, dans laquelle l'aldéhyde est du glutaraldéhyde.

9. Composition adhésive selon l'une quelconque des revendications précédentes, dans laquelle le mélange est stérile.

10. Composition adhésive médicale stérile selon l'une quelconque des revendications 1 à 9, comprenant un mélange d'une protéine globulaire et d'un aldéhyde polyfonctionnel ou d'un dialdéhyde, dans laquelle l'adhéslf, lorsqu'll a durci, présente une résistance au déchirement au moins égale à 75 g/cm², tel que mesuré grâce au Test d'Adhérence des Tissus.

11. Trousse comprenant un premier moyen formant conteneur présentant une protéine plasmatique ou une protéine globulaire disposées à l'intérieur de celui-ci, et un second moyen formant conteneur présentant un aldéhyde polyfonctionnel ou un dialdéhyde disposés à l'intérieur de celui-ci pour être utilisés dans l'administration thérapeutique d'un adhésif médical comprenant un mélange d'une protéine plasmatique ou d'une protéine globulaire et d'un aldéhyde polyfonctionnel ou d'un dialdéhyde.

12. Trousse selon la revendication 11, dans laquelle la protélne est de l'albumine.

13. Trousse comprenant un premier moyen formant conteneur présentant une composante protéinique plasmatique disposée à l'intérieur de celui-ci, à une concentration comprise entre 30 % et 55 % en polds, et un second moyen formant conteneur comprenant une composante polyaidéhydique ou dialdéhydique disposée à l'intérieur de celui-ci à une concentration comprise entre 5 % et 15 % en poids, les composantes protéinique et aldéhydique étant présentes en des quantités suffisantes pour permettre le mélange d'une partie en poids de la composante aldéhydique avec entre 5 et 60 parties en poids de la composante protéinique pour être utilisées dans l'administration thérapeutique d'un adhésif médical comprenant un melange d'une protéine plasmatique ou d'une protéine globulaire et d'un aldéhyde polyfonctionnel ou d'un dialdéhyde.

14. Applicateur permettant de déposer une composition adhésive sur la surface d'un tissu et présentant une protéine plasmatique ou une protéine globulaire et un aldéhyde polyfonctionnel ou un dialdéhyde disposés à l'intérieur de celui-ci, dans lequel l'applicateur comprend un premier et un second compartiment séparé, la protéine étant disposée à l'intérieur du premier compartiment et l'aldéhyde étant disposé à l'intérieur du second compartiment de l'applicateur.

15. Applicateur selon la revendication 14, dans laquelle l'applicateur est une seringue.

16. Composition adhésive selon l'une quelconque des revendications 1 à 10 pour être utilisée dans un procédé de liaison d'un tissu biologique avec une substance comprenant le tissu à mettre en contact avec la substance.

17. Adhésif selon là revendication 16, dans lequel la substance est un tissu biologique.

18. Adhésif selon les revendications 16 ou 17, dans lequel la substance est une matière synthétique.

19. Adhésif selon les revendications 16, 17 ou 18, dans lequel la protéine et l'aldéhyde sont appliqués de maniére séquentielle sur la surface et sont mélangés *in situ* pour former la composition.

20. Adhésif selon les revendications 16, 17 ou 18, dans lequel la protéine et l'aldéhyde sont mélangés pour former la composition avant l'application de celle-ci sur la surface du tissu.

21. Utilisation d'une composition comprenant un mélange d'une protéine plasmatique ou d'une protéine globulaire et d'un aldéhyde polyfonctionnel ou d'un dialdéhyde pour fabriquer un adhésif médical.

22. Utilisation selon la revendication 21. dans laquelle la protéine est de l'albumine.

23. Utilisation selon les revendications 21 ou 22, dans laquelle l'adhésif, lorsqu'il a durci, présente une résistance au déchirement au molns égale à 75 g/cm², tel que mesuré grâce au Test d'Adhérence des Tissus.

24. Utilisation selon la revendication 23, dans laquelle la résistance au déchirement est au moins égale à 400 g/cm².

25. Utilisation selon l'une quelconque des revendications 21 à 24, dans laquelle la composante protéinique est présente à une concentration comprise entre 27 % et 53 % en poids, par rapport au poids de la composition.

26. Utilisation selon l'une quelconque des revendications 21 à 25, dans laquelle la composante aldéhydique est présente à une concentration comprise entre 0,5 % et 5 % en poids, par rapport au poids de la composition.

27. Utilisation selon l'une quelconque des revendications 21 à 26, dans laquelle la composante aldéhydique est un dialdéhyde choisi parmi le groupe composé de glyoxal, succinaldéhyde, glutaraldéhyde, maléaldéyde et phtalaldéhyde.

28. Utilisation selon la revendication 27, dans laquelle l'aldéhyde est du glutaraldéhyde.

29. Utilisation selon l'une quelconque des revendications 21 à 28, dans laquelle le mélange est stérile.

30. Utilisation selon les revendications 21 à 29, comprenant un mélange d'une protéine globulaire et d'un aldéhyde polyfonctionnel ou d'un dialdéhyde. dans laquelle l'adhésif, lorsqu'il a durci, présente une résistance au déchirement au moins égale à 75 g/cm², tel que mesuré grâce au Test d'Adhérence des Tissus.

31. Utilisation selon l'une quelconque des revendications 21 à 30 pour être utilisée dans un procédé de liaison d'un tissu biologique avec une substance comprenant le tissu à mettre en contact avec la substance.

32. Utilisation selon la revendication 31, dans laquelle la substance est un tissu biologique.

33. Utilisation selon les revendications 31 ou 32, dans laquelle la substance est une matière synthétique.

34. Utilisation selon les revendications 31, 32 ou 33, dans laquelle la protéine et l'aldéhyde sont appliqués de manière séquentielle sur la surface et sont mélangés *in situ* pour former la composition.

35. Utilisation selon les revendications 31, 32 ou 33, dans laquelle la protéine et l'aldéhyde sont mélangés pour former la composition avant l'application de celle-ci sur la surface du tissu.
